# EUROPÄISCHE PATENTSCHRIFT

(11) **EP 0 792 859 B1**
(45) Veröffentlichungstag und Bekanntmachung des Hinweises auf die Patenterteilung: **20.09.2000**
(21) Anmeldenummer: 97102627.3
(22) Anmeldetag: 19.02.1997
(51) Int. Cl.: C07C 29/04, B01J 21/06

(54) **Verwendung von Katalysatoren auf Basis von Siliziumdioxid enthaltenden Katalysatorträgern für katalytische Umsetzungen unter hydrothermalen Bedingungen**
Use of catalysts based on silica containing catalyst carriers for catalytic reactions under hydrothermal conditions
Utilisation des catalyseurs à base de supports de catalyseurs contenant du silice pour réactions catalysées hydrothermales

(30) Priorität: 28.02.1996 DE 19607494
(43) Veröffentlichungstag der Anmeldung: 03.09.1997
(73) Patentinhaber: Degussa-Hüls Aktiengesellschaft, 60287 Frankfurt am Main (DE)
(72) Erfinder: Lansink-Rotgerink, H.G.J., Dr., 63864 Glattbach (DE); Riedemann, Heike, 63776 Mömbris (DE); Tacke, Thomas, Dr., 61381 Friedrichsdorf (DE); Krause, Helmfried, 63517 Rodenbach (DE); Freund, Andreas, Dr., 63801 Kleinostheim (DE); Burmeister, Roland, Dr., 63826 Geiselbach (DE); Panster, Peter, Dr., 63517 Rodenbach (DE)

(56) Entgegenhaltungen:
- EP-A- 0 393 356
- EP-A- 0 503 229
- EP-A- 0 578 441

## Beschreibung

Die Erfindung betrifft die Verwendung von Katalysatoren, welche eine Aktivkomponente auf einem Siliziumdioxid enthaltenden Katalysatorträger aufweisen, für katalytische Reaktionen unter hydrothermalen Bedingungen. Hydrothermale Bedingungen liegen bei chemischen Umsetzungen in wäßrigen Systemen vor, wenn bei Temperaturen oberhalb des Siedepunktes von Wasser und bei Drucken oberhalb von Normaldruck gearbeitet wird.

Eine typische Umsetzung unter hydrothermalen Bedingungen ist die Hydratisierung von Olefinen zu den entsprechenden Alkoholen in Gegenwart von Phosphorsäure als Katalysator beziehungsweise Aktivkomponente auf einem Siliziumdioxid enthaltenden Katalysatorträger.

Ein solches Verfahren wird zum Beispiel in der EP 0 578 441 A2 beschrieben. Nach diesem Verfahren wird Wasser und Ethylen bei Temperaturen zwischen 225 und 280°C und Drucken zwischen 20 und 240 bar zu Ethanol umgesetzt. Dabei wird ein Wasser/Ethylen-Molverhältnis im Bereich von 0,15 bis 0,5 angewendet. Die Katalysatorbelastung, gemessen in Gramm Wasser/Ethylen-Gemisch pro Minute und Milliliter Katalysator, kann im Bereich von 0,01 bis 0,1 g/(min × ml) gewählt werden. Als Nebenprodukt wird bei dieser Reaktion Diethylether gebildet.

Die Herstellung von Isopropanol durch Hydratisierung von Propylen erfolgt unter ähnlichen Bedingungen wie oben Bereich zwischen 180 und 225°C. Als Nebenprodukt entsteht bei dieser Reaktion n-Propanol.

Als Katalysatorträger für die Aktivkomponente Phosphorsäure verwendet die EP 0 578 441 A2 Pellets aus synthetischem Siliziumdioxid mit hoher Bruchfestigkeit, hoher Porosität und geringen metallischen Verunreinigungen. Die Poren des Trägers dienen zur Aufnahme der Aktivkomponente. Das Porenvolumen ist daher bevorzugt größer als 0,8 ml/g. Der mittlere Porenradius vor dem Einsatz im Hydratisierungsprozeß liegt im Bereich zwischen 1 und 50 nm.

Zur Erzielung einer optimalen Leistung bei der Hydratisierung wird von der EP 0 578 441 A2 ein Gehalt des Trägers an Siliziumdioxid von wenigstens 99 Gew.-% vorgeschrieben mit Verunreinigungen unter 1 Gew.-%, bevorzugt unter 0,3 Gew.-%.

Katalysatoren unterliegen im Betrieb einer Alterung, die sich durch eine Verringerung der Aktivität und/oder Selektivität bemerkbar macht. Die Desaktivierung beruht häufig auf einer durch hohe Temperaturen verursachten Verringerung der spezifischen Oberfläche des Trägers.

Die spezifische Oberfläche des Trägers ist eng mit seiner Porenstruktur verbunden. Darüber hinaus weisen hochoberflächige Feststoffe meist eine vollständig amorphe oder überwiegend amorphe Struktur auf, welche das Bestreben hat, unter Kristallitwachstum und Verringerung der spezifischen Oberfläche in einen thermodynamisch stabilen Zustand überzugehen.

Es hat sich gezeigt, daß auch Siliziumdioxid enthaltende Katalysatorträger einer solchen Alterung unterliegen. Hydrothermale Bedingungen beschleunigen die Alterung. Weiterhin ist es bekannt, daß auch Verunreinigungen, insbesondere durch Alkalimetalle, die Alterung von Siliziumdioxid enthaltenden Trägern unter hydrothermalen Bedingungen fördern (siehe zum Beispiel R.K. Iler in "The Chemistry of Silica", Seite 544, John Wiley & Sons (1979)).

Überraschenderweise hat sich gezeigt, daß auch die in der EP 0 393 356 beschriebenen Katalysatorträger auf Basis von pyrogen hergestelltem Siliziumdioxid unter hydrothermalen Bedingungen einer Alterung unterliegen, wobei kleine Poren zu größeren Poren unter Verlust von spezifischer Oberfläche zusammenwachsen. Das Porenvolumen ändert sich dabei anfänglich kaum. Diese Alterung ist unerwartet, da das pyrogene Siliziumdioxid, aus denen die Träger bestehen, eine hervorragende Temperaturbeständigkeit aufweisen. Beim Erhitzen auf Temperaturen bis zu 1000°C für die Dauer von 7 Tagen ändert pyrogenes Siliziumdioxid nach Untersuchungen mit einem Rasterelektronenmikroskop seine Morphologie nicht (Schriftenreihe Pigmente Nr. 11: Grundlagen von AEROSIL®; Firmenschrift der DEGUSSA, 5. Auflage, Juni 1993, Seite 20).

Aufgabe der vorliegenden Erfindung ist es daher, Siliziumdioxid enthaltende Katalysatorträger anzugeben, welche bei Verwendung unter hydrothermalen Bedingungen eine verbesserte Alterungsstabilität aufweisen.

Diese Aufgabe wird gelöst durch die Verwendung von Katalysatoren, welche eine Aktivkomponente auf einem Siliziumdioxid enthaltenden Katalysatorträger aufweisen, wobei der Katalysatorträger durch Imprägnieren mit löslichen Verbindungen von mindestens einem Element der Gruppe IVB des Periodensystems der Elemente modifiziert ist.

Als Siliziumdioxid kann ein natürliches oder synthetisches Material verwendet werden. Als natürliches Siliziumdioxid kommen zum Beispiel Bentonite wie Montmorillonit in Frage.

Von den synthetischen Materialien sind naßchemisch hergestellte Kieselgele oder Fällungskieselsäuren geeignet. Bevorzugt werden durch Flammenhydrolyse hergestellte, sogenannte pyrogene Siliziumdioxide, verwendet.

Die genannten Materialien werden in an sich bekannter Weise zu Katalysatorformkörpern verarbeitet. Geeignete Formkörper aus pyrogenem Siliziumdioxid werden zum Beispiel in der EP 0 393 356 beschrieben. Sie weisen bei Außendurchmessern von 2 bis 15 mm eine spezifische Oberfläche von 50 bis 400 m²/g, ein Porenvolumen von 0,6 bis 1,3 ml/g sowie eine Bruchfestigkeit von 50 bis 150 N auf. Ihr Abrieb ist kleiner als 1 %. Die Preßlinge bestehen zu 99 Gew.-% aus Siliziumdioxid und haben ein Schüttgewicht zwischen 400 und 500 g/l. Sie weisen keine Poren mit Durchmessern unter 5 nm auf.

Die Porenstruktur der aus den Siliziumdioxidmaterialien geformten Katalysatorträger wird durch Imprägnieren mit löslichen Verbindungen von Elementen der Gruppe IVB des Periodensystems der Elemente stabilisiert. Hierzu werden die Katalysatorträger mit mindestens einem Element der Gruppe IVB in einer Menge von 0,1 bis 25, bevorzugt von 0,5 bis 10 Gew.-%, bezogen auf das Gesamtgewicht des Trägers, imprägniert. Unterhalb von 0,1 Gew.-% wird keine ausreichende Stabilisierung der Porenstruktur erhalten. Abhängig von dem verwendeten Siliziumdioxidmaterial und den zur Anwendung kommenden hydrothermalen Bedingungen kann es notwendig sein, die Katalysatorträger mit wenigstens 0,5, bei aggressiveren Reaktionsbedingungen mit wenigstens 1 Gew.-%, zu imprägnieren.

Die Katalysatorträger werden bevorzugt nach dem Verfahren der Porenvolumenimprägnierung mit den stabilisierenden Elementen belegt. Dieses Verfahren gestattet eine definierte Beladung der Katalysatorträger. Andere Imprägnierverfahren sind jedoch ebenfalls anwendbar.

Zur Porenvolumenimprägnierung werden die löslichen Verbindungen der Stabilisierungselemente in einem Lösungsmittelvolumen gelöst, welches gleich dem Porenvolumen der vorgelegten Katalysatorträger ist und anschließend zum Beispiel durch Sprühen über die Träger verteilt, die zur Erzielung einer gleichmäßigen Imprägnierung während des Sprühvorganges in einem Dragierkessel umgewälzt werden können.

Zur Imprägnierung können sowohl wäßrige als auch organische Lösungsmittel oder Mischungen davon verwendet werden. Die geeignete Auswahl hängt von der verwendeten Verbindung der Stabilisierungselemente ab. Bei Verwendung von Titan(III)-chlorid (TiCl₃) zur Stabilisierung mit Titan wird Wasser als Lösungsmittel verwendet. Anstelle von Titan(III)-chlorid kann auch eine organische Titanverbindung wie zum Beispiel Tetrabutoxytitan (Ti(C₄H₉O)₄) eingesetzt werden. In diesem Falle eignet sich Butanol als Lösungsmittel.

Nach der Imprägnierung wird der Katalysatorträger getrocknet, wobei im Falle organischer Lösungsmittel zur Vermeidung einer Explosionsgefahr entsprechende Sicherheitsmaßnahmen ergriffen werden müssen. Daran anschließend können die behandelten Träger bei Temperaturen zwischen 160 und 800°C calciniert werden.

Die erfindungsgemäße Verwendung ist besonders vorteilhaft für die Hydratisierung von Olefinen. Die Stabilisierung des Trägers ist jedoch auch bei anderen katalytischen Umsetzungen unter hydrothermalen Bedingungen günstig.

Im Falle der Hydratisierung von Olefinen wird Phosphorsäure als Aktivkomponente in den Katalysatorträger eingebracht.

Hierzu wird der Träger nach der Stabilisierung und nach der gegebenenfalls abschließenden Calcinierung in eine wäßrige Lösung von Phosphorsäure getaucht und mit dieser getränkt. Zur Anwendung kommen hierbei Phosphorsäurelösungen mit 15 bis 85 Gew.-% Phosphorsäure bezogen auf das Gesamtgewicht der Lösung.

Ein Hauptanwendungsgebiet der Hydratisierung von Olefinen ist die Hydratisierung von Ethylen zur Herstellung von Ethanol und Diethylether sowie die Hydratisierung von Propylen zur Herstellung von Isopropanol. Es werden dabei die aus dem Stand der Technik bekannten Reaktionsbedingungen angewendet.

Im folgenden wird die Änderung der Porenstruktur von Siliziumdioxid enthaltenden Katalysatorträgern unter hydrothermalen Bedingungen untersucht. Es werden konventionelle Träger mit stabilisierten Trägern verglichen.

Es zeigen
- Figur 1:: Porenstruktur eines unstabilisierten Katalysator trägers nach hydrothermalem Alterungstest.
- Figur 2:: Porenstruktur eines mit 1,5 % Titan stabilisierten Katalysatorträgers nach hydrothermalem Alterungstest.
- Figur 3:: Porenstruktur eines mit 4 % Titan stabilisierten Katalysatorträgers nach hydrothermalem Alterungstest.
- Figur 4:: Porenstruktur eines mit 5 % Titan stabilisierten Katalysatorträgers nach hydrothermalem Alterungstest.

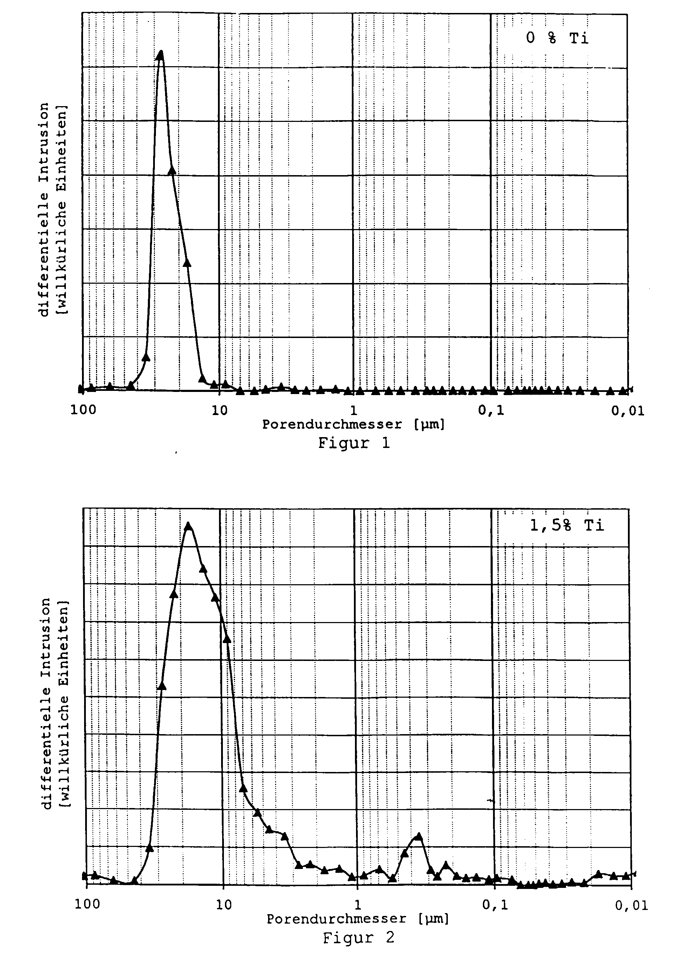

Die in den Figuren 1 bis 4 gezeigten Porenverteilungskurven wurden mit Hilfe der bekannten Hg-Porosimetrie ermittelt. Sie geben die differentielle Eindringung (Intrusion) des Quecksilbers in Abhängigkeit vom Porendurchmesser wieder. Für die differentielle Intrusion wurden willkürliche Einheiten gewählt und die Kurven jeweils über den zur Verfügung stehenden Diagrammbereich gedehnt.

### Vergleichsbeispiel 1

Ein Katalysatorträger aus pyrogener Kieselsäure (AEROSIL®-Träger 350 von Degussa; spezifische Oberfläche 180 m²/g; Schüttdichte 490 g/l; gesamtes Porenvolumen 0,8 cm³/g; Tabletten von 6 mm Durchmesser und 5,5 mm Höhe) wurde mit Phosphorsäure (60 Gew.-%)beladen und 41 Stunden in einer Hochdruckanlage bei einem Wasserdampfdruck von 15 bar auf 350°C geheizt. Die Porenverteilung des gealterten Katalysators wurde mit Hg-Porosimetrie bestimmt. Die gemessene Porenverteilung ist in Figur 1 grafisch dargestellt.

Die hydrothermal gealterten Träger weisen ein Maximum der Porenverteilung bei Porendurchmessern zwischen 20 und 30 µm auf.

### Beispiel 1

Der oben erwähnte Katalysatorträger wurde mit 1.5 Gew-% Ti modifiziert. Zur Modifizierung von 100 g Träger mit 1,5 Gew.-% Titan wurden 33 g einer 15 %igen Titanchlorid-Lösung (TiCl₃) mit Wasser auf 80 ml entsprechend dem Porenvolumen des Trägermaterials verdünnt. Mit dieser Lösung wurde das Trägermaterial imprägniert.

Nach 30 Minuten Einwirkzeit wurde der Träger bei 100°C während 3 Stunden im Trockenschrank getrocknet und dann in einem Ofen bei 600°C für die Dauer von 4 Stunden calciniert. Anschließend wurde der Träger mit Phosphorsäure (60 Gew.-%) beladen und 40 Stunden in einer Hochdruckanlage bei einem Wasserdampfdruck von 15 bar und 350°C belassen. Die Porenverteilung des gealterten Katalysators wurde wiederum mit Hg-Porosimetrie bestimmt. Die Porenverteilung ist in Figur 2 grafisch dargestellt.

Das Maximum in der Porenverteilung liegt zwischen 10 und 20 µm. Im Vergleich zu dem im Vergleichsbeispiel 1 eingesetzten undotierten Katalysator, weist der mit 1,5 Gew.-% Ti dotierte Katalysator auch nach Alterung einen größeren Anteil an kleinen Poren mit einem Durchmesser unterhalb von 10 µm auf.

### Beispiel 2

Ein Katalysatorträger wie in Vergleichsbeispiel 1 beschrieben wurde mit 4 Gew-% Ti modifiziert. Zur Modifizierung von 100 g Träger mit 4 Gew.-% Titan wurden 85,93 g einer 15 %igen Titanchlorid-Lösung mit Wasser auf 80 ml verdünnt und zur Imprägnierung des Trägers über den Träger verteilt.

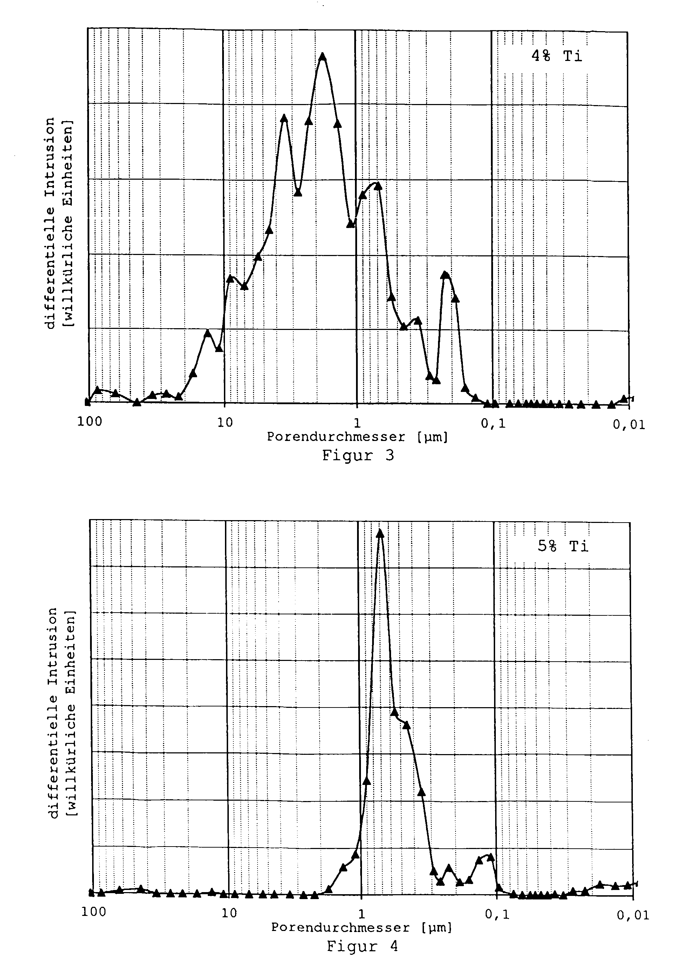

Nach 30 Minuten Einwirkzeit wurde der Träger bei 100°C während 3 Stunden im Trockenschrank getrocknet und dann in einem Ofen bei 600°C für die Dauer von 4 Stunden calciniert. Anschließend wurde der Träger mit Phosphorsäure (60 Gew.-%) beladen und 43 Stunden in einer Hochdruckanlage bei einem Wasserdampfdruck von 15 bar und 350°C belassen. Die Porenverteilung des gealterten Katalysators wurde mit Hg-Porosimetrie bestimmt. Die Porenverteilung ist in Figur 3 grafisch dargestellt.

Die Porenverteilung dieser Probe ist sehr breit. Das Maximum in der Porenverteilung liegt bei ca. 2 µm. Im Vergleich zu dem im Vergleichsbeispiel 1 eingesetzten undotierten Katalysator weist der mit 4 Gew-% Ti dotierte Katalysator einen sehr großen Anteil an Poren mit einem Durchmesser kleiner 10 µm auf. Im Vergleich zu dem undotierten Katalysator aus Vergleichsbeispiel 1 ist der mit 4 Gew-% Ti dotierte Katalysator deutlich stabiler, die Vergrößerung des Porendurchmessers ist deutlich weniger ausgeprägt.

### Beispiel 3

Ein Katalysatorträger wie in Vergleichsbeispiel 1 beschrieben wurde mit 5 Gew-% Ti modifiziert. Zur Modifizierung von 100 g Träger mit 5 Gew.-% Titan wurden 35,5 g Tetrabutoxytitan (Ti(C₄H₉O)₄) mit Butanol auf 80 ml verdünnt und über den Träger verteilt.

Nach 30 Minuten Einwirkzeit wurde der Träger bei 100°C während 3 Stunden im Trockenschrank getrocknet und dann in einem Ofen bei 600°C für die Dauer von 4 Stunden calciniert. Anschließend wurde der Träger mit Phosphorsäure beladen und 41,5 Stunden in einer Hochdruckanlage bei einem Wasserdampfdruck von 15 bar auf 350°C geheizt. Die Porenverteilung des gealterten Katalysators wurde mit Hg-Porosimetrie bestimmt. Das Ergebnis ist in Figur 4 grafisch dargestellt.

Das Maximum in der Porenverteilung liegt bei ca. 0.7 µm. Es gibt nahezu keine Poren mit einem Durchmesser größer 3 µm. Im Vergleich zu dem undotierten Katalysator aus Vergleichsbeispiel 1 ist der mit 5 Gew-% Ti dotierte Katalysator deutlich stabiler. Der mittlere Porendurchmesser ist für den mit 5 Gew-% Ti dotierten Katalysator um den Faktor 35 kleiner als im Falle des undotierten Katalysators aus Vergleichsbeispiel 1.

Diese Beispiele belegen eindeutig die sehr gute Langzeitstabilität der Ti-dotierten Phosphorsäure-Katalysatoren auf Basis von Siliziumdioxid enthaltenden Katalysatorträgern.

Eine Analyse des Porenvolumens der Träger zeigte, daß sich das Porenvolumen durch die hydrothermale Alterung nur unwesentlich änderte. Es fand lediglich ein Zusammenwachsen der kleineren Poren des frischen Trägers zu größeren Poren statt, wobei dieses Wachstum durch die Stabilisierung verlangsamt werden konnte.

## Patentansprüche

1. Verwendung von Katalysatoren, welche eine Aktivkomponente auf einem Siliziumdioxid enthaltenden Katalysatorträger aufweisen, für katalytische Umsetzungen unter hydrothermalen Bedingungen,
**dadurch gekennzeichnet,**
daß der Katalysatorträger durch Imprägnieren mit löslichen Verbindungen von mindestens einem Elemente der Gruppe IVB des Periodensystems der Elemente modifiziert ist.

2. Verwendung nach Anspruch 1,
**dadurch gekennzeichnet,**
daß der Katalysatorträger mit mindestens einem Element der Gruppe IVB des Periodensystem in einer Menge von 0,1 bis 25 Gew.-% bezogen auf das Gewicht des Trägers modifiziert ist.

3. Verwendung nach Anspruch 2 für die Hydratisierung von Olefinen.

4. Verwendung nach Anspruch 3,
**dadurch gekennzeichnet,**
daß als Aktivkomponente Phosphorsäure verwendet wird.

5. Verwendung nach Anspruch 4 für die Hydratisierung von Ethylen zur Herstellung von Ethanol und Diethylether.

6. Verwendung nach Anspruch 4 für die Hydratisierung von Propylen zur Herstellung von Isopropanol.

## Claims

1. Use of catalysts, which have an active component on a catalyst support containing silicon dioxide, for catalytic reactions under hydrothermal conditions,
characterised in that
the catalyst support is modified by impregnation with soluble compounds of at least one element from group IVB of the periodic system of elements.

2. Use according to claim 1,
characterised in that
the catalyst support is modified with at least one element from group IVB of the periodic system in a quantity of 0.1 to 25 wt.%, relative to the weight of the support.

3. Use according to claim 2 for hydrating olefins.

4. Use according to claim 3,
characterised in that
phosphoric acid is used as the active component.

5. Use according to claim 4 for hydrating ethylene to produce ethanol and diethyl ether.

6. Use according to claim 4 for hydrating propylene to produce isopropanol.

## Revendications

1. Utilisation de catalyseurs qui possèdent un composant actif sur un support de catalyseur contenant du dioxyde de silicium, pour des réactions catalytiques dans des conditions hydrothermales,
caractérisée en ce que
le support de catalyseur est modifié par imprégnation avec des composés solubles d'au moins un élément du groupe IVB du système périodique des éléments.

2. Utilisation selon la revendication 1,
caractérisée en ce que
le support de catalyseur est modifié avec au moins un élément du groupe IVB du système périodique, en une quantité de 0,1 à 25 % en poids rapporté au poids du support.

3. Utilisation selon la revendication 2 pour l'hydratation des oléfines.

4. Utilisation selon la revendication 3,
caractérisée en ce qu' on utilise comme composant actif de l'acide phosphorique.

5. Utilisation selon la revendication 4 pour l'hydratation de l'éthylène en vue de la production d'éthanol et d'éther diéthylique.

6. Utilisation selon la revendication 4 pour l'hydratation du propylène en vue de la production d'isopropanol.
